(19) Europäisches Patentamt
European Patent Office
Office européen des brevets

(11) **EP 2 262 361 B1**

(12) **FASCICULE DE BREVET EUROPEEN**

(45) Date de publication et mention de la délivrance du brevet:
**23.03.2016 Bulletin 2016/12**

(21) Numéro de dépôt: **08787889.8**

(22) Date de dépôt: **02.04.2008**

(51) Int Cl.:
***A01K 67/02*** (2006.01)

(86) Numéro de dépôt international:
**PCT/FR2008/000450**

(87) Numéro de publication internationale:
**WO 2009/122019 (08.10.2009 Gazette 2009/41)**

(54) **PROCÉDÉ DE PRODUCTION MASSIVE DE CHROMOBOTIA MACRACANTHUS**

MASSENPRODUKTIONSVERFAHREN FÜR CHROMOBOTIA MACRACANTHUS

MASS PRODUCTION METHOD FOR CHROMOBOTIA MACRACANTHUS

(84) Etats contractants désignés:
**AT BE BG CH CY CZ DE DK EE ES FI FR GB GR HR HU IE IS IT LI LT LU LV MC MT NL NO PL PT RO SE SI SK TR**

(43) Date de publication de la demande:
**22.12.2010 Bulletin 2010/51**

(73) Titulaires:
• **Institut De Recherche Pour Le Développement I.R.D.**
**13572 Marseille Cedex 02 (FR)**
• **Agency For Marine And Fisherie Research And Development, The Ministry of Marine Affairs and Fisheries, the Republic of Indonesia**
**Jakarta 14430 (ID)**

(72) Inventeurs:
• **SLEMBROUCK, Jacques**
**Jakarta (ID)**
• **LEGENDRE, Marc**
**F-34980 Saint-Clement De Riviere (FR)**
• **POUYAUD, Laurent**
**F-34270 Le Triadou (FR)**

• **SATYANI, Darti**
**Jakarta Selatan**
**12540-DK1 Jakarta (ID)**
• **SUGAMA, ketut**
**Jakarta Selatan 12540-DK1 Jakarta (ID)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
• **JOURNAL OF APPLIED ICHTYOLOGY, vol. 15, 1999, pages 49-53, XP002515280 BLACKWELL WISSENSCHAFTS-VERLAG, BERLIN**
• **NEWTON CASTAGNOLI, EDWARD M.DONALDSON: "induced Ovulation And Rearing Of The Pacu (Colossoma Mitrei)" AQUACULTURE, vol. 25, 1981, pages 275-279, XP002515281 Elsevier Scientific Publishing Company, Amsterdam**
• **SCHIAVONE R., ZILLI L., VILELLA S., FAUVEL C.: AQUACULTURE, no. 255, 2006, pages 522-531, XP025041092**

EP 2 262 361 B1

**Description**

La présente invention a pour objet un procédé de production massive de poissons de l'ordre des Cypriniformes, notamment de la famille des Cobitidae, en particulier de *Chromobotia macracanthus.*

En 1996, la valeur d'exportation des poissons et invertébrés d'ornement a dépassé 200 millions de dollars E.-U, dont plus de 60 pour cent (soit quelque 130 millions de dollars) ont été aux économies des pays en développement. Depuis 1985, le commerce international en organismes aquatiques d'ornement augmente au rythme annuel moyen de 14 pour cent. Les organismes capturés à l'état sauvage ne représentent qu'un faible pourcentage du commerce total d'ornement. Mais c'est cet aspect de l'industrie qui aura probablement le plus grand impact sur les communautés de pêcheurs des pays en développement.

L'Asie et l'Amérique du Sud sont les principaux fournisseurs de poissons d'ornement dans le monde alors que les Etats-Unis, l'Europe et le Japon sont les principaux importateurs. En 1998, l'industrie des poissons d'aquarium en Asie du SUD-EST était estimée entre 150 et 200 millions de dollars E.-U et Singapour, de loin le plus gros exportateur, vendait entre 80 et 90 millions de dollars E.-U. Ces chiffres sont sous évalués et l'on parle de plus de 300 à 400 millions de dollars E.-U uniquement pour l'Asie du SUD-EST. La production de Singapour est basée en grande partie sur le reconditionnement et la revente de poissons provenant des pays voisins comme l'Indonésie, la Malaisie, Ceylan et la Thaïlande.

Bien qu'une partie de la production indonésienne passe par Singapour, l'exportation des organismes aquatiques à partir de l'Indonésie est officiellement de 13,4 millions de dollars E.-U, ce chiffre plaçant l'Indonésie au 4ème rang des exportateurs mondiaux en 2004. Cependant, la valeur donnée par les quarantaines des aéroports indonésiens est de 130 millions de dollars E-U. D'après le Ministère des Affaires Maritimes et de la Pêche, ce pays possède 48 000 petits et gros producteurs de poissons d'ornement d'eau douce qui produisent plus de 60 millions de poissons par an. Parmi ces producteurs, un grand nombre attend avec impatience une technique de production fiable de *Chromobotia macracanthus.*

*Chromobotia macracanthus* (Bleeker) est classé dans la « catégorie 1 » de l'industrie des poissons d'ornement d'eau douce de l'Asie du Sud-Est. Originaire d'Indonésie et capturé dans les rivières de Sumatra et de Kalimantan, C. *macracanthus* est exporté vers tous les continents. Vendu entre 6 à 8 ∈ (voir plus) en Europe, le marché international était estimé en 1998 à 20 millions d'individus mesurant 3 à 8 cm exportés d'Indonésie. Il semble que ce chiffre soit fortement sous-estimé et la demande internationale augmente régulièrement. Ces estimations ne portent que sur les effectifs exportés et ne tiennent pas compte des pertes après récolte (maladies, stress, etc.) ou des dommages causés (habitat, autres espèces aquatiques capturées de façon annexe). Les acteurs de la filière constatent une baisse régulière des captures de juvéniles qui font l'objet d'une pêche d'intensité croissante depuis presque 30 ans. Cette espèce est manifestement surexploitée et menacée, ce qui a amené le gouvernement indonésien à interdire, par décret en 2002, l'exportation de poissons de plus de 15 cm considérés comme sexuellement matures. Ce décret a pour objectif de protéger les géniteurs dans leur environnement naturel.

Si au cours des prochaines décennies, la demande internationale n'est pas satisfaite par des juvéniles issus d'élevage, *C. macracanthus* disparaîtra avec son industrie, alors que l'exploitation de ce poisson au niveau local est une source importante de revenus pour les communautés rurales.

De plus, au niveau international, les acteurs sont de plus en plus concernés et contraints par les normes internationales sur la protection de l'environnement et plus de 80% des espèces actuellement commercialisées proviennent d'élevage.

La maîtrise du cycle de production de cette espèce est donc un enjeu international et la reproduction artificielle de *C. macracanthus* en captivité semble avoir été obtenue occasionnellement en Thaïlande, en Malaisie, en République tchèque, en Russie et en Floride, mais aucun rapport ou article ne le confirme, ni ne présente de support technique correspondant.

En 2005, bien que les instituts de recherches indonésiens tentaient de maîtriser le cycle de reproduction de *C. macracanthus* depuis plus de 10 ans (Satyani D. (2004) Journ. Penel. Perik. Indon. 10(5), p.55-59 ; Satyani D., H. Mundriyanto, T. Kadarini dan Kusdiarti 2005 Prosiding Seminar Nasional dan Konggres Biologi XIII. Fakultas Biologi UGM. Yogyakarta, p. 435-438 ; Subandiyah S. (2005) Prosiding Seminar Nasional dan Konggres Biologi XIII. Fakultas Biologi UGM. Yogyakarta p. 290-293 ; Subandiyah S. dan D. Satyani (2004) Prosiding Seminar Biologi menuju Millenium III. Fakultas Biologi UGM. Yogyakarta p. 127-136), aucun itinéraire technique de production n'avait pu être établi. Des travaux de recherches sur la maturation en milieu naturel, sur la stimulation et l'amélioration de la maturation sexuelle en captivité, ont été effectués en utilisant des implants hormonaux, en testant des aliments enrichies ou naturels, en faisant varier la photopériode Satyani D., Chumaidi dan Kusdiarti. (2006) Pros. Sem. Nas. Pengembangan Tekn. Budidaya Perikanan dan Temu Bisnis Kerapu. Balai Besar Riset Perik. Budidaya Laut Gondol dan Balai Riset Perik. Air Payau, Maros. PRPB-BRKP. Bali. 111 ; Subandiyah S. (2005) Prosiding Seminar nasional Perikanan Indonesia. STP. Jakarta p.185-192 ; Chumaidi. (2005) Prosiding Sem Nasional Tahunan Hasil Penel. Perik. dan Kelautan. UGM. Yogyakarta p. 249-254 ; Effendi I., T. Prasetya, A.O. Sudrajat, N. Suhenda et K. Sumawidjaja (2003) Jurnal Akuakultur Indonesia, 2(2), p. 51-54 ; Satyani D., T. Kadarini dan O. Komarudin (1999) Joum. Penel. Perik. Indon. II (4), p. 37-42 ; Subagja J., O. Komarudin dan J. Effendi. (1997) Jurn. Penel. Perik. Indon. III (2), p.10 - 17).

Toutefois à l'issue de ces investigations, les géniteurs de *C. macracanthus* maintenus en captivité atteignent toujours avec difficultés leur pleine maturation sexuelle. Celle-ci est aléatoire, de courte durée et dépend des fluctuations climatiques. Bien que des oeufs fécondés aient parfois été obtenus après induction hormonale de l'ovulation, les résultats sont restés peu reproductibles et sans aucune production significative d'alevins. Le cycle de production est toujours un échec et la quantité d'ovules collectée est plus faible que celle obtenue en milieu naturel. Les taux de fécondation sont très faibles, plus de 70% des oeufs meurent en court d'incubation et 100% des larves meurent entre 5 et 9 jours après éclosions.

Aussi existe-t-il un besoin important de mise à disposition d'un procédé de production de Juvéniles de *C. macracanthus* qui soit fiable, reproductible et qui permette d'obtenir des taux de fécondation élevés ainsi que de forts taux de survie des oeufs et des larves.

Au cours de leurs travaux, les Inventeurs ont trouvé des conditions d'élevages qui permettent de résoudre l'ensemble de ces problèmes et ont mis au point un procédé fiable, reproductible et qui permet d'obtenir des taux de fécondation élevés ainsi que de forts taux de survie des oeufs et des larves.

Aussi la présente invention a-t-elle pour objet un procédé de production massive de poissons de l'ordre des Cypriniformes, notamment de la famille des Cobitidae en structure d'élevage en circuit fermé comprenant les étapes suivantes :

a) élevage dans la pénombre des géniteurs mâles et femelles à une densité comprise entre 5 et 15 poissons par $m^3$, avantageusement entre 6 à 7 poissons par $m^3$, dans un bac d'élevage alimenté en eau dont la température est maintenue entre 30 et 32 °C pendant une période comprise entre 6 et 15 mois, avantageusement pendant 14 mois puis dont la température est diminuée à 25-29 °C, avantageusement 25-26 °C et maintenue à cette valeur pendant une période comprise entre 1 et 6 mois, avantageusement 3 mois, les géniteurs étant alimentés pendant toute la durée d'élevage le matin avec des aliments composés distribués à raison de 0,5 à 1 % de la biomasse des poissons et le soir avec des vers de terre représentant au moins 10 % de la biomasse des poissons, avantageusement 10 à 15 % de ladite biomasse,

b) sélection des génitrices femelles présentant un diamètre ovocytaire compris entre 1,00 et 1,32 mm,

c) sélection des géniteurs mâles chez lesquels le sperme s'écoule bien avec un massage abdominal (stade 3 de la maturité sexuelle),

d) traitement hormonal des génitrices femelles,

- soit par deux injections d'une combinaison de gonadolibérine purifiée ou sous forme d'analogues synthétiques et d'un composé anti-dopaminergique, lesdites injections étant séparées par un délai de 5 à 8 heures, avantageusement égal à 6 heures, les doses pour la première injection étant comprises entre 2 et 10 $\mu$g/kg, avantageusement égales à 8 $\mu$g/kg pour la gonadolibérine et ses analogues synthétiques et comprises entre 1 et 5 mg, avantageusement égales à 4 mg pour le composé antidopaminergique et les doses pour la deuxième injection étant comprises entre 8 et 12 $\mu$g/kg, avantageusement égales à 12 $\mu$g/kg pour la gonadolibérine purifiée ou sous forme d'analogues synthétiques et comprises entre 4 et 6 mg, avantageusement égales à 6 mg pour le composé antidopaminergique,

- soit par une première injection d'hormone gonadotropine chorionique humaine (hCG) à raison de 250 à 500 UI/kg, avantageusement à raison de 500 UI/kg suivie, 18 heures à 26 heures après, d'une seconde injection d'une combinaison de gonadolibérine purifiée ou sous forme d'analogue synthétique et d'un composé anti-dopaminergique à une dose comprise entre 4 et 12 $\mu$g/kg, avantageusement égale à 12 $\mu$g/kg pour la gonadotropine purifiée ou sous forme d'analogues synthétiques et à une dose comprise entre 2 et 6 mg/kg, avantageusement égale à 6 mg/kg pour le composés antidopaminergique,

e) traitement hormonal des géniteurs mâles par une injection d'une combinaison de gonadolibérine purifiée ou sous forme d'analogue synthétique et d'un composé anti-dopaminergique à une dose comprise entre 2 et 10 $\mu$g/kg, avantageusement égale à 8 $\mu$g/kg pour la gonadotropine purifiée ou sous forme d'analogues synthétiques et à une dose comprise entre 1 et 5 mg/kg, avantageusement égale à 4 mg/kg pour le composés antidopaminergique,

f) collecte des ovules par massage abdominal chez les génitrices femelles qui s'effectue avec un délai de latence moyen (délai entre injections et collecte des ovules) variable en fonction de la température de l'eau de stabulation des géniteurs durant la période de latence entre le traitement hormonal et l'ovulation (entre 24 et 31 °C, mais avantageusement entre 26 et 28°C) et de la nature du traitement hormonal utilisé, selon les relations suivantes :

- pour le traitement avec deux injections successives de gonadolibérine associée à un antidopaminergique :

$$Y \text{ (Temps de latence en heure)} = 413,97 - 29,47\, X + 0,54\, X^2 \ (R^2 = 0,670),$$

avec X= température en degrés Celsius
- pour le traitement avec une injection de hCG suivie d'une injection de gonadolibérine associée à un antidopaminergique :

$$Y \text{ (Temps de latence en heure)} = 771,51 - 54,08\, X + 0,96\, X^2 \ ; \ (R^2 = 0,652),$$

avec X = température en degrés Celsius

g) collecte du sperme des géniteurs mâles 10 à 48 h après traitement hormonal, avantageusement 12 à 24 h, directement dans une solution d'immobilisation des spermatozoïdes, avantageusement une solution saline (NaCl 0,9%), et conservation au frais, avantageusement à 4 °C,

h) fécondation *in vitro* des oeufs,

i) incubation des oeufs sous légère agitation à une température comprise entre 24 et 28 °C, avantageusement à 26 °C jusqu'à éclosion,

j) recueil des larves,

k) élevage des larves jusqu'au stade juvénile.

L'ordre des Cypriniformes comprend la famille des Balitoridae, la famille des Catostomidae, la famille des Cobitidae, la famille des Cyprinidae, la famille des Gyrinocheilidae et la famille des Psilorhynchidae. Dans un mode de réalisation avantageux de l'invention, le procédé concerne l'élevage des Cobitidae, en particulier les *Chromobotia macracanthus,* issus notamment des populations de Sumatra et de Bornéo (Kalimantan).

On entend par pénombre des conditions dans lesquelles la luminosité est comprise entre 0 et 20 Lux, avantageusement entre 5 et 10 Lux ; ainsi les géniteurs peuvent être élevés en bassins partiellement recouverts d'un couvercle ou dans une enceinte fermée avec un éclairage rouge de type laboratoire de tirage photographique.

Selon l'invention, les poissons sont soumis le matin à une alimentation classique connue de l'homme du métier ; ils peuvent être notamment nourris 6 jours par semaine le matin avec des aliments composés comprenant par exemple entre 35 et 45 % de protéines et entre 5 et 15 % de lipides; le choix de l'alimentation faisant partie des connaissances de l'homme du métier, celui-ci est à même d'adapter cette alimentation si nécessaire. L'apport de vers de terre le soir est un élément essentiel pour l'obtention de bons géniteurs.

Dans les étapes b) et c) du procédé selon l'invention, les poissons sont avantageusement placés dans des structures de stabulation de petite taille (par ex. bacs de 150 à 300 L) pour faciliter leur manipulation pendant le traitement hormonal puis lors de la collecte des gamètes,

A la fin de l'étape f), les ovules son conservés jusqu'à la fécondation dans des conditions classiques, notamment pendant une période n'excédant pat 10 minutes à une température comprise entre 25 et 28 °C.

La conservation des spermatozoïdes au frais à la fin de l'étape g) permet une utilisation différée de quelques heures.

Dans un mode de réalisation avantageux du procédé de l'invention les analogues synthétiques de la gonadolibérine sont choisis dans le groupe comprenant GnRHa (avantageusement D-Arg6, Trp7, Leu8, Pro9, Net) ou LHRH-A et le composé antidopaminergique dans le groupe comprenant le dompéridone et le pimozide.

Dans un autre mode avantageux de réalisation du procédé selon l'invention l'eau utilisée est de l'eau recyclée circulant à un débit compris entre 80% et 100%, de renouvellement en eau par heure du volume d'élevage. L'eau utilisée présente avantageusement un pH compris entre 7,0 et 8,0, une conductivité comprise entre 100 et 300 µS/cm et sa concentration en oxygène est comprise entre 6,0 et 8,0 mg/L.

Dans un autre mode de réalisation avantageux du procédé selon l'invention, l'étape d'incubation des oeufs de l'étape i) est réalisée dans des conditions d'agitation modérée obtenues soit par agitation des oeufs dans un courant d'eau, avantageusement dans un incubateur en forme d'entonnoir comprenant une arrivée d'eau par le fond dans la partie étroite de l'entonnoir, ladite arrivée d'eau présentant un diamètre compris entre 20 et 30 mm pour permettre un débit d'entrée d'eau compris entre 0,3 L.mn$^{-1}$ et 2,3 L.mn$^{-1}$, soit dans des récipients en eau non renouvelée placés sur un plateau oscillant avec 56 à 88 oscillations par minute autour d'un angle d'une amplitude totale de 10 degrés (+5, -5). On entend par agitation modérée une agitation suffisamment forte pour maintenir les oeufs en mouvement mais suffisamment faible pour ne pas induire d'anomalie de développement ni de rupture du chorion.

Ainsi le procédé selon l'invention comprend 3 étapes fondamentales :

1- une phase de maturation progressive des gonades ou phase de construction qui peut s'effectuer à une température de l'eau relativement élevée, avantageusement comprise entre 30 et 32°C,

2- une phase de régression de la maturation sexuelle, si les poissons sont maintenus en eau chaude (≥ 30°C) sur une période prolongée,

3- une stimulation du développement des gonades (vers l'achèvement de la vitellogenèse) par la baisse de la température à 25-26°C.

Outre la température de l'eau, la gestion de l'alimentation (apport très abondant en vers de terre) et le maintien d'une bonne qualité de l'eau sont des facteurs déterminants de la bonne santé des géniteurs et de leur aptitude à effectuer leur maturation gonadique.

Le procédé selon l'invention présente les avantages suivants :

1. des systèmes et conditions d'élevage des géniteurs, notamment des géniteurs de *C. macracanthus* permettant une survie élevée et une bonne maturation sexuelle de ces poissons en captivité ;

2. des critères objectifs pour la sélection des géniteurs aptes à répondre au traitement d'induction de la reproduction

3. des traitements hormonaux efficaces permettant d'induire la maturation finale des ovocytes et l'ovulation chez les femelles, et de stimuler la spermiation des mâles ;

4. une définition des temps de réponse aux traitements hormonaux et des protocoles de collecte et de conservation des ovules et des spermatozoïdes ;

5. des modalités fiables d'incubation des oeufs après fécondation artificielle, permettant l'obtention de pourcentages d'éclosion élevés, avec une forte proportion de larves viables,

6. des conditions d'élevage des larves permettant d'obtenir des taux de survie élevés après 1 mois, âge auquel les poissons sont placés en structure de pré-grossissement.

L'obtention d'un taux d'éclosion moyen peu élevé, voire nul, après induction de l'ovulation, demeurait l'un des problèmes majeurs pour le contrôle de la reproduction de *C. macracanthus* en élevage. En effet jusqu'en 2006, malgré l'obtention d'un taux d'ovulation et d'un pourcentage d'oeufs fécondés satisfaisants, le taux d'éclosion moyen était généralement très faible (26,5% ± 25,2%) à l'issue de la phase d'incubation des oeufs,avec une proportion de larves anormales très importante, en moyenne 57,6 ± 25,2 %.

Depuis, les recherches réalisées par les inventeurs sur cette phase clé de la maîtrise de la reproduction de *C. macracanthus* en captivité ont démontré l'importance de deux facteurs principaux sur la survie des oeufs et des embryons : l'agitation des oeufs et la température de l'eau d'incubation.

Les exemples 1 à 5 et les figures 1 à 9 qui suivent illustrent l'invention.

La figure 1 représente l'évolution, en fonction de la température de l'eau, du pourcentage de femelles en cours de vitellogénèse (stade 3) et en fin de vitellogénèse (stade 4) de *C. macracanthus* provenant de Sumatra (**A**) et de Kalimantan (**B**) maintenues en captivité en circuit fermé dans les conditions de l'exemple 1.

La figure 2 représente l'évolution, en fonction de la température de l'eau, du pourcentage de mâles en cours de maturation sexuelle (stade 1) et de males (stade 2 et 3) de *C. macracanthus* provenant de Sumatra (**A**) et de Kalimantan (**B**) maintenus en captivité en circuit fermé.

La figure 3 représente l'évolution moyenne du diamètre modal ovocytaire de 2 femelles de *C. macracanthus* isolées à 25-26°C et de 2 autres à 30-31 °C. Après un élevage dans un même bassin en eau recyclée à 30-31 °C, les quatre femelles sont isolées dans des bacs identiques (250 L) thermorégulés aux températures choisies.

La figure 4 représente le pourcentage d'ovulation obtenu après traitement hormonal selon l'invention en fonction du diamètre modal initial des ovocytes. Les résultats sont les résultats globaux des deux types de traitements hormonaux.

La figure 5 représente la relation entre le temps de latence après traitement hormonal et la température de l'eau de stabulation des géniteurs de *Chromobotia macracanthus.* A : femelles traitées avec deux injections successives de Ovaprim® ; B : femelles traitées avec une injection de hCG suivie d'une injection de Ovaprim® (Rond clairs : femelles originaires de Sumatra ; ronds sombres : femelles originaires de Kalimantan ; Triangles : femelles de Sumatra pour lesquelles le processus de maturation ovocytaire a été engagé suite à la première injection de Ovaprim®)

La figure 6 représente l'influence de l'agitation des oeufs sur la qualité de l'incubation des oeufs chez *C. macracan-*

*thus.*
La figure 7 représente un modèle d'incubateur en forme d'entonnoir et en fibre de verre.
La figure 8 représente l'influence de la température d'incubation sur le pourcentage d'éclosion (A) et la proportion de larves viables chez *C. macracanthus (B).*
La figure 9 représente le développement des larves de *Chromobotia macracanthus* de l'éclosion à l'âge de 31 jours.

**Exemple 1 : Conditions d'élevage et maturation sexuelle des géniteurs**

**1.1. Conditions d'élevage**

Afin de pouvoir étudier les paramètres influents sur la maturation sexuelle de C. *macracanthus* en captivité, des structures d'élevage en circuit fermé adaptées à la maintenance de cette espèce ont été conçues et réalisées sur la station de Depok (Indonésie). Des géniteurs de *C. macracanthus* provenant de Sumatra (N=66 ; 70% de Femelles) et un groupe provenant de Kalimantan (N=59 ; 75% de femelles) ont été mis en élevage à une densité de 6 à 7 poissons par m$^3$.
Chaque bac d'élevage (10 m$^3$) est alimenté en eau recyclée (débit= 8,2 m$^3$.h$^{-1}$) à l'aide d'un filtre mécanique, d'un filtre biologique et d'une lampe U.V. (stérilisation). De façon à diminuer le stress, les poissons sont isolés dans une pièce sombre. Cette pièce isolée thermiquement permet aussi de réguler la température tout au long de l'année à un niveau choisi entre 25°C et 32°C. Chaque circuit fermé est alimenté en eau de forage. Sursaturée en gaz, cette eau doit être préalablement aérée pendant 24 h avant d'être utilisée pour l'élevage. Les valeurs mensuelles de température de l'eau ont fluctué entre 30 et 32°C pendant 14 mois puis la température a été ajustée à 26 et 27°C pendant 6 mois.
Les poissons sont alimentés 6 jours par semaine ; le matin des aliments artificiels granulés sont distribués à raison de 0,5 à 1% de la biomasse et le soir la distribution se fait avec des lombrics en fonction de la demande (apports > 10% de la biomasse des poissons). Les bassins d'élevage sont siphonnés 2 fois par semaine avec un apport hebdomadaire d'eau neuve de 10%.

**1.2. Résultats**

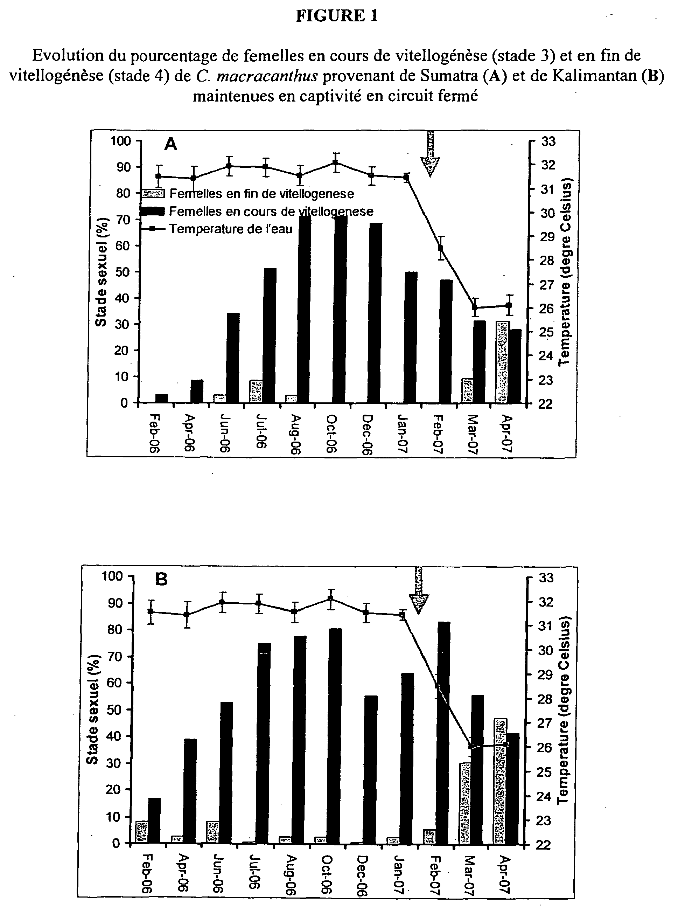

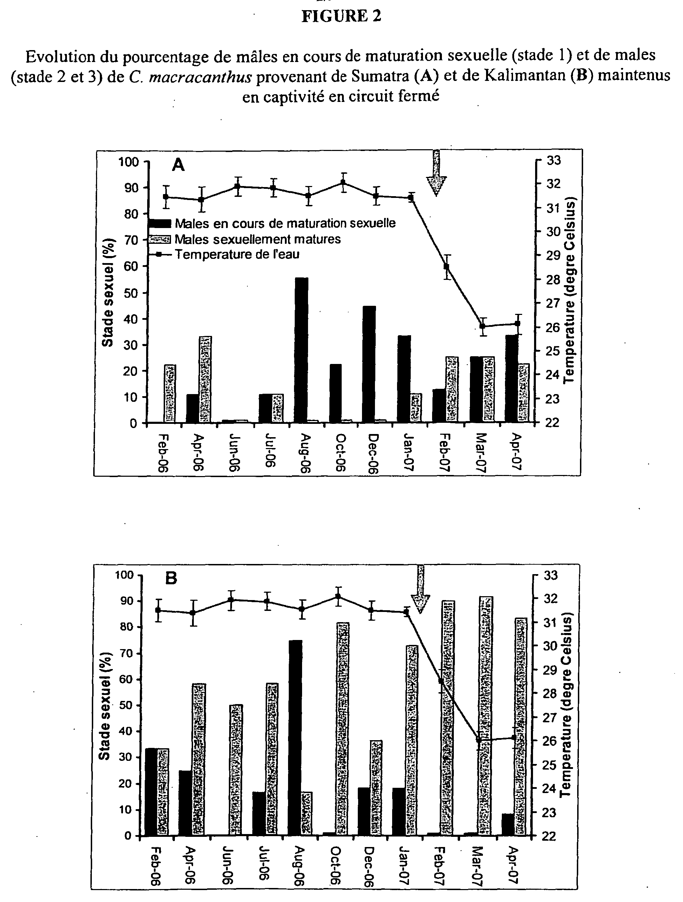

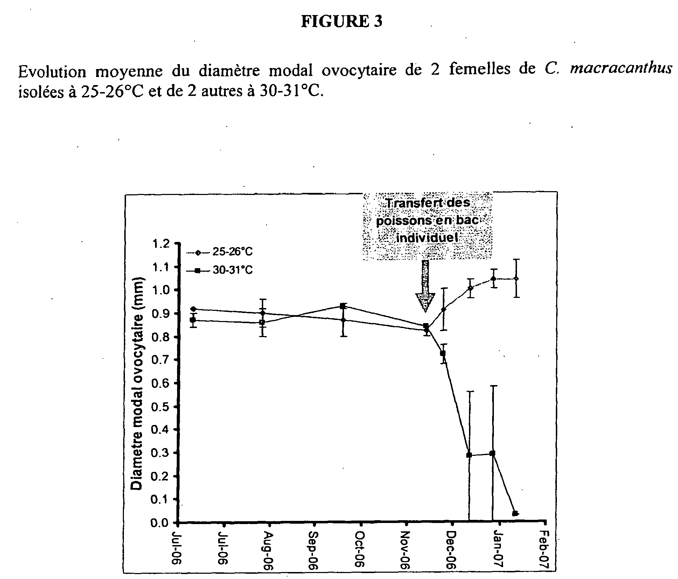

Ils sont illustrés dans les figures 1 à 3.
Après 20 mois d'élevage, les principaux paramètres physico-chimiques de l'eau des structures d'élevage sont restés en dessous des normes toxiques pour les géniteurs de *C. macracanthus ;* le pH est compris entre 7,1 et 7,7 ; la conductivité varie de 114 à 294 μS/cm et la concentration en oxygène relevée montre peu de variation (6,7-8,1 mg.L$^{-1}$). La teneur en ammoniac non ionisé est comprise entre 0,0 et 0,1 mg.L$^{-1}$, alors que les concentrations varient entre 0,00 et 0,03 mg.L$^{-1}$ pour les nitrites et entre 6,3 et 20,0 mg.L$^{-1}$ pour les nitrates.
La pratique d'alimentation utilisée est très bien adaptée aux besoins de cette espèce puisque les femelles présentent de l'embonpoint et sont en pleine maturation sexuelle (évaluation réalisée selon le mode opératoire décrit dans l'exemple 2). Dans ces conditions d'élevage on obtient jusqu'à 90% de femelles en cours de maturation avec 40-47% de femelles prêtes à être reproduites. Aucune pathologie n'a été constatée dans ces élevages et la survie est restée supérieure à 85%.
La chute de température volontaire de l'eau d'élevage a permis d'obtenir 80-90% des femelles en cours de maturation sexuelle après 2 mois de basse température (26-27°C). Suite à la chute de température provoquée, la proportion de femelles prêtes à pondre est passée de 8 à 50% pour les poissons de la population originaire de Kalimantan et de 9% à 30% pour celle provenant de Sumatra (Figure 1).
Dans ces conditions d'élevage, on obtient entre 60 et 90% de mâles sexuellement matures, c'est-à-dire au stade 3 (Figure 2). Ces observations ont été effectuées entre avril et juillet, soit en saison opposée à la période de reproduction naturelle qui se concentre entre septembre et décembre dans les rivières de Sumatra.
Parallèlement, quatre femelles de même stade sexuel, après élevage dans un même bassin en eau recyclée à 30-31°C, sont isolées dans des bacs identiques (250 L) thermorégulés aux températures choisies de 30°C ou 26°C. Après 2 mois d'observation, les ovocytes des femelles placées à température froide ont montré une croissance régulière et sont en fin de vitellogénèse (aptitude à la reproduction) tandis que les ovocytes des femelles placées à température chaude ont complètement régressé (Figure 3).

**Exemple 2 : Critères de sélection des géniteurs pour le traitement d'induction de la reproduction**

**2.1. Mode opératoire**

Afin de faciliter le suivi individuel, chaque géniteur est anesthésié (bain contenant 0,3 mL.L$^{-1}$ de Phenoxy-2-éthanol) puis marqué avec une marque électronique (PIT Tag) insérée dans la musculature dorsale à l'aide d'un trocart.

Des échantillonnages sont effectués tous les 45-60 jours, la condition des géniteurs (rapport poids corporel/longueur[3]) et leur maturation sexuelle sont évaluées. Lors de chaque échantillonnage, tous les poissons sont pêchés, identifiés, mesurés et pesés individuellement, ceci afin de déterminer la prise de poids mensuelle et de réévaluer les rations alimentaires.

Le stade de la maturation sexuelle de chaque femelle est évalué en prélevant une cinquantaine d'ovocytes par biopsie intra-ovarienne à l'aide d'un cathéter souple (diamètre externe : 2 mm). Les ovocytes sont placés dans une solution saline (NaCl 0,9%) et leur plus grand diamètre est mesuré immédiatement à l'aide d'une loupe binoculaire (x25) afin de déterminer le diamètre modal du groupe d'ovocyte le plus développé. On considère quatre stades de maturation sexuelle principaux :

Stade 1 : femelles immatures (non canulables).
Stade 2 : ovaire avec ovocytes en pré-vitellogénèse (diamètre modal $\leq$ 0,4 mm).
Stade 3 : Développement de l'ovaire et phase de forte croissance ovocytaire, présence de nombreux ovocytes en phase de vitellogénèse exogène (0,4 mm < diamètre modal < 1,02 mm).
Stade 4 : présence d'une grande majorité (70 - 90%) d'ovocytes postvitellogéniques (diamètre ovocytaire modal $\geq$ 1,02 mm), aptes à ovuler après traitement hormonal.

Le stade de maturité sexuelle des mâles est déterminé en fonction de la présence et de la quantité de sperme émise après massage abdominal des poissons selon l'échelle arbitraire suivante :

Stade 0 : absence de sperme
Stade 1 : émission d'une faible quantité de sperme avec une pression abdominale soutenue,
Stade 2 : présence de sperme en quantité moyenne, émission aisée lors du massage abdominal,
Stade 3 : sperme abondant, s'écoule avec une légère pression abdominale.

## 2.2. Résultats

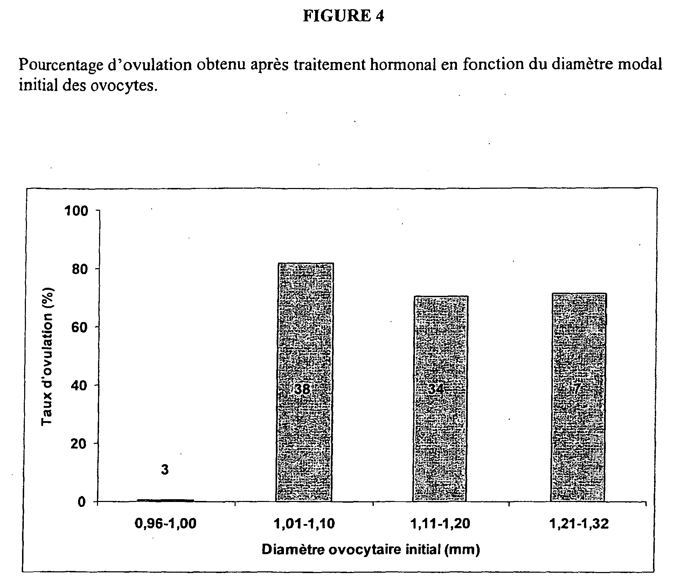

Ils sont donnés dans la figure 4 et le tableau 1.

Après biopsie intra-ovarienne, les femelles utilisées pour la reproduction induite ont été sélectionnées sur la base de leur embonpoint et d'un diamètre ovocytaire modal $\geq$ 1,02 mm, diamètre ovocytaire modal minimal à partir duquel l'ovulation peut être induite avec succès chez *C. macracanthus* après traitement hormonal. (Figure 4). L'embonpoint des femelles (aspect rebondi du ventre) est un critère de second rang qui permet d'affiner la sélection des femelles pour la reproduction. Il est évalué selon une échelle subjective à 3 niveaux : 0- pas d'embonpoint particulier de la femelle, 1- abdomen notablement rebondi, 2-abdomen très rebondi. De fait, les femelles avec un abdomen rebondi conduisent à l'obtention d'une quantité d'ovules significativement plus élevée que celles qui ne présente pas d'embonpoint marqué (p<0,05) et le taux d'ovulation tend également à être plus élevé (p>0,05). En revanche, la qualité des ovules, évaluée par le taux de fécondation, ne montre pas de différence marquée entre les deux groupes de femelles (Tableau 1).

Tableau 1 : Comparaison des réponses ovulatoires et des quantités (fécondité) et qualités (fécondation) des ovules collectés après traitement hormonal (avec injection préparatoire de hCG) chez les femelles de *C. macracanthus* en fonction de leur embonpoint initial (moyenne $\pm$ sd).

| Embonpoint | Réponse ovulatoire (%) | Fécondité (n/kg femelle) | Fécondation (%) | Effectif |
|---|---|---|---|---|
| Absent | 56 | 54 576 $\pm$ 29 240 | 63 $\pm$ 31 | 9 |
| Notable ou fort | 70 | 120 730 $\pm$ 38 250 | 61 $\pm$ 16 | 10 |

Le diamètre ovocytaire modal, déterminé après biopsie intraovarienne, permet donc d'évaluer l'aptitude des ovocytes à répondre au traitement hormonal appliqué pour obtenir l'ovulation. L'embonpoint de la femelle sert quant à lui d'indicateur de la quantité d'ovules qui pourra être collectée après ovulation. Utilisé seul, ce second critère ne serait cependant pas suffisamment fiable car l'embonpoint peut avoir aussi d'autres causes que le développement de la gonade (quantité de graisse périviscérale, prise alimentaire, notamment).

**Exemple 3 : Traitements hormonaux et gestion des gamètes collectés**

**3.1. Mode opératoire**

Comme la grande majorité des poissons téléostéens, *Chromobotia macracanthus* ne se reproduit pas spontanément en captivité (quelques pontes ont cependant été observées en aquarium), bien qu'il soit capable d'y effectuer sa maturation gonadique. Il est donc indispensable de recourir à un traitement hormonal pour induire la maturation finale des ovocytes et l'ovulation. Chez les mâles, le traitement hormonal permet d'augmenter la quantité de sperme collecté et de faciliter cette collecte du fait d'une réduction de la viscosité de la semence.

Pour les femelles, deux traitements hormonaux ont montré leur efficacité. Le premier traitement consiste en deux injections d'Ovaprim®, séparées par un délai de 6 h, et à des doses respectives de 0,4 puis 0,6 mL/kg. Le second traitement correspond à une première injection de hCG (Gonadotropine Chorionique Humaine) à 500 UI.kg$^{-1}$ suivie 24 h plus tard d'une injection d'Ovaprim® à 0,6 mL/kg. Pour ces deux traitements, le temps de latence entre la seconde injection d'hormone et l'ovulation (détectée par la possibilité de collecter les ovules par massage abdominal des femelles) a été comparé pour différentes températures de stabulation des poissons.

Les mâles sont choisis au stade 3 de la maturité sexuelle et ne reçoivent qu'une injection d'Ovaprim® à une dose de 0,4 mL/kg. Le sperme, collecté par massage abdominal 16 à 24 h après le traitement hormonal, est recueilli directement dans une seringue contenant une solution de NaCl 155 mM (1 volume pour 4 volume) de façon à éviter une possible activation des spermatozoïdes due à une contamination du sperme par de l'urine, puis conservé à 5°C avant d'être utilisé pour la fécondation.

Une fois l'ovulation détectée (à cette fin, chaque femelle est examinée plusieurs fois à 1 ou 2 h d'intervalle), les ovules sont collectés par massage abdominal, puis pesés et fécondés avec du sperme provenant de plusieurs mâles.

La qualité des ovules de chaque femelle est évaluée par les taux de fécondation et d'éclosion obtenus à partir de lots expérimentaux répliqués, constitués d'environ 150 oeufs incubés dans de petits récipients en plastiques d'une capacité en eau de 300 mL. La procédure de fécondation in vitro de ces lots d'oeufs expérimentaux est la suivante : environ 150 ovules sont placés dans un récipient en plastique, 100 µL de sperme dilué dans la solution saline sont ajoutés sur les ovules puis la motilité des spermatozoïdes est activée par l'adition de 10 mL d'eau. Une fois l'eau ajoutée, les oeufs sont agités délicatement durant 1 minute pour permettre la fécondation. L'eau et le sperme sont ensuite éliminés et les oeufs rincés avec de l'eau propre avant d'être placés en incubation. Toutes les incubations expérimentales ont été réalisées en eau de source, dans des conditions standardisées. Pour les essais de production massive de larves, le restant des oeufs de chaque femelle est fécondé puis incubé en eau recyclée en écloserie.

L'efficacité de ces traitements hormonaux a été évaluée sur 62 femelles ayant effectué leur maturation sexuelle dans de bonnes conditions tant en termes d'alimentation que de qualité de l'eau.

Les poissons utilisés appartiennent à 3 groupes distincts :

- 39 femelles, originaires de Sumatra, qui ont été capturées à pleine maturité sexuelle directement dans la rivière Musi (province de Sumatra Sud) puis stockées temporairement en cages flottantes. Leur reproduction a été induite moins de 10 jours après leur capture, soit sur place en bord de rivière, soit après transport par avion et voiture jusqu'à la station expérimentale de Depok. Ce groupe correspond donc à des individus ayant effectué leur maturation gonadique en conditions naturelles en rivière.
- 14 femelles, appartenant à la même population que la précédente (rivière Musi, Sumatra) mais placées en élevage en circuit fermé thermorégulé à la station de Depok pendant plus d'une année avant les essais de reproduction induite, et
- 9 femelles originaires d'une population différente, en provenance de l'île de Bornéo (province indonésienne de Kalimantan), et placées en élevage en circuit fermé thermorégulé, dans les mêmes conditions que les femelles provenant de Sumatra.

**3.2. Résultats**

A noter également que pour les poissons maintenus en condition d'élevage en circuit fermé, la pénombre et l'attention des expérimentateurs ont permis de limiter les facteurs de stress des poissons

Les résultats obtenus démontrent l'efficacité des traitements hormonaux choisis pour induire l'ovulation. Aucune différence significative n'a été mise en évidence entre les deux traitements hormonaux utilisés, que ce soit en termes de réponse ovulatoire obtenue ou de quantité et de qualité des ovules collectés.

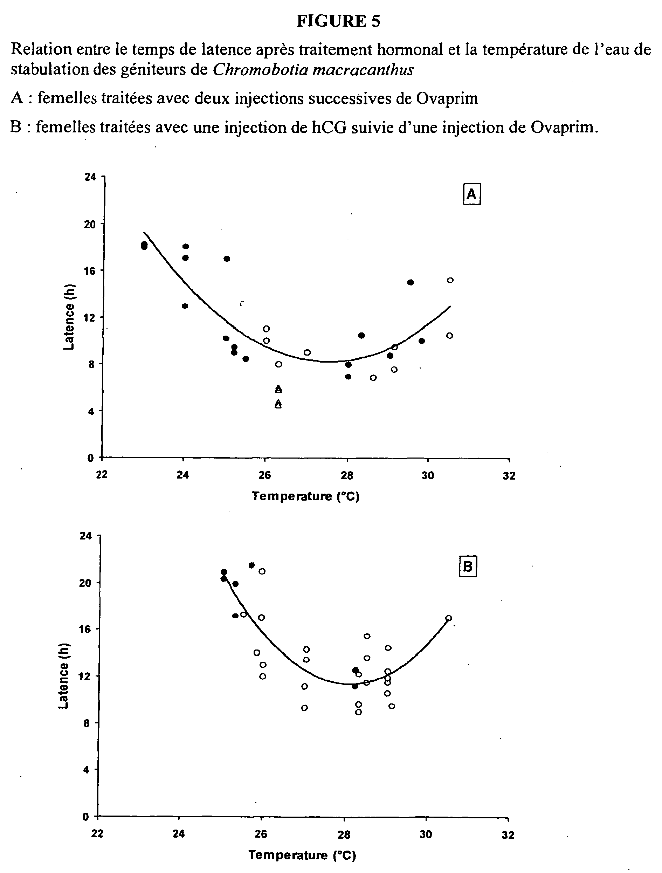

L'évolution du temps de latence (temps séparant la seconde injection hormonale de l'ovulation) en fonction de la température de stabulation des géniteurs, testée entre 23°C et 30,5°C, montre une relation tout à fait inhabituelle chez les poissons. En effet, cette relation généralement proche de la linéarité (le temps de latence diminuant avec la température selon une relation log-linéaire) est ici de forme polynomiale du second degré quel que soit le traitement hormonal considéré (Figure 5). Les relations sont très hautement significatives pour tous les termes des relations. Les équations correspondantes sont les suivantes (Y= temps de latence en h et X = T = température en °C) :

Traitement à deux injections successives d'Ovaprim® :

$$Y = 413,97 - 29,47\,X + 0,54\,X^2 \; ; \; R^2 = 0,670$$

Traitement à une injection de hCG suivi d'une injection d'Ovaprim® :

$$Y = 771,51 - 54,08\,X + 0,96\,X^2 \; ; \; R^2 = 0,652$$

Des informations relatives à l'étude de l'écologie de l'espèce en milieu naturel, auxquelles s'ajoutent les études expérimentales de l'effet de la température sur le succès de l'incubation des oeufs indiquent que la plage de température se situant aux alentours de 26-28°C est optimale pour la reproduction de l'espèce. A cette température, le temps de latence est d'environ 4 h plus long avec le traitement hCG + Ovaprim® en comparaison du traitement Ovaprim® + Ovaprim®. A mesure que l'on s'éloigne de cette plage thermique vers les basses ou les hautes températures, le temps de latence tend à augmenter rapidement. Vers les basses températures, l'augmentation du temps de latence n'est pas surprenante puisqu'elle correspond au moins pour partie à un ralentissement du métabolisme chez ces organismes poïkilothermes. En revanche, l'augmentation du temps de latence vers les températures plus élevées est paradoxale puisque l'on devrait attendre une accélération du métabolisme et donc une réduction du temps de latence comme cela a été rapporté chez de nombreuses espèces de poissons téléostéens. Les mécanismes physiologiques qui sous-tendent cette réponse paradoxale n'ont pas encore été identifiés et doivent faire l'objet d'étude complémentaire. Il ne semble pas cependant que l'espèce se reproduise naturellement dans des eaux de températures supérieures à 28,0°C puisque cette reproduction serait vouée à l'échec du fait de la mortalité des embryons.

Les taux d'ovulation obtenus, compris entre 78 et 82%, sont systématiquement élevés pour les femelles des trois populations testées (Tableau 2).

Les quantités d'ovules collectées sont également élevées et correspondent à un ratio moyen de 8%, 9% et 6% du poids des femelles, pour les deux populations de Sumatra et celle de Kalimantan respectivement. Ces rapports gonado-somatiques moyens élevés indiquent une réponse ovulatoire complète induite par les traitements hormonaux. En d'autres termes, la quasi totalité des ovocytes postvitellogéniques présents dans l'ovaire effectuent leur maturation ovocytaire et ovulent suite au traitement hormonal. Des cas d'ovulation partielle n'ont été suspectés qu'en de très rares occasions. Les fécondités relatives correspondantes (nombre d'ovules collectés rapporté au poids de la femelle) sont de l'ordre de 10 500 ovules pour une femelle de 100 g de masse corporelle issue de la population de Sumatra. Il est très important de souligner ici que la fécondité des femelles de Sumatra élevées en circuit fermé se situe au même niveau que celle des femelles ayant effectué leur maturation gonadique en conditions naturelles en rivière. Ceci démontre que les conditions définies ci avant pour la maintenance des géniteurs en captivité permettent d'atteindre la pleine maturité sexuelle des femelles. Bien que la différence ne soit pas statistiquement significative, on note que la fécondité tend à être plus faible pour les femelles appartenant à la population originaire de Kalimantan (tableau 2).

La qualité des ovules collectés, évaluée par le pourcentage de fécondation, est tout à fait satisfaisante pour les femelles appartenant à la population de Sumatra (de 61 à 74% en moyenne). La qualité des ovules pour les femelles originaires de la population de Kalimantan est moins bonne (34% en moyenne).

Tableau 2 : Réponse ovulatoire, fécondité relative et pourcentages de fécondation obtenus après traitement hormonal pour trois populations de *C. macracanthus.*

| Origine et Conditions de maturation des femelles | Effectif | Réponse ovulatoire (%) | Fécondité (n.kg$^{-1}$) | Fécondation (%) |
|---|---|---|---|---|
| Sumatra Rivière | 39 | 82 | 103 550 ± 14 125 | 61 ± 5 |
| Sumatra Circuit fermé | 14 | 79 | 108 670 ± 14 090 | 74 ± 9 |
| Kalimantan Circuit fermé | 9 | 78 | 73 780 ± 11 240 | 34 ± 7 |

**Exemple 4 : Modalités d'incubation des oeufs après fécondation artificielle**

**4.1. Mode opératoire**

*4.1.1. Agitation des oeufs*

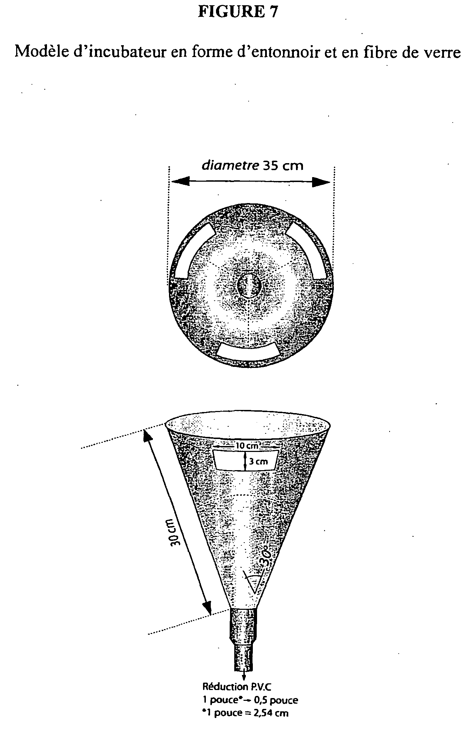

Environ 150 oeufs sont soit placés dans des récipients en plastique de 300 mL en eau posés sur un plateau oscillant permettant de maintenir les oeufs en mouvement permanent, soit mis dans un courant d'eau permettant un mouvement des oeufs lent et sans choc. Bien que d'autres types d'incubateurs soient également fonctionnels, des incubateurs en forme d'entonnoir avec une arrivée d'eau par le fond, permettant de maintenir les oeufs en mouvement dans un courant d'eau modéré ont été utilisés. Des points importants dans ce type de système sont une surface de matériaux suffisamment lisse pour ne pas endommager les oeufs et un diamètre de l'arrivée d'eau suffisamment large pour avoir un flux d'eau efficace sans un débit trop important à l'arrivée dans l'incubateur. L'application d'un débit trop fort peut en effet conduire à l'éclatement prématuré du chorion de l'oeuf et à la mort des embryons en développement. Des incubateurs de dimensions indiquées en figure 7 ont été testés et permettent de bons résultats avec un débit d'eau en entrée de 0,3 L.mn$^{-1}$ et 2,3 L.mn$^{-1}$L.m$^{-1}$.

Parallèlement des oeufs sont incubés dans les mêmes conditions de température mais en eau stagnante.

*4.1.2. Influence de la température d'incubation*

L'incubation est conduite à différentes températures comprises entre 22°C et 30°C, à savoir 22 °C, 24 °C, 26 °C, 28 °C et 30 °C.

**4.2. Résultats**

*4.2.1. Agitation des oeufs*

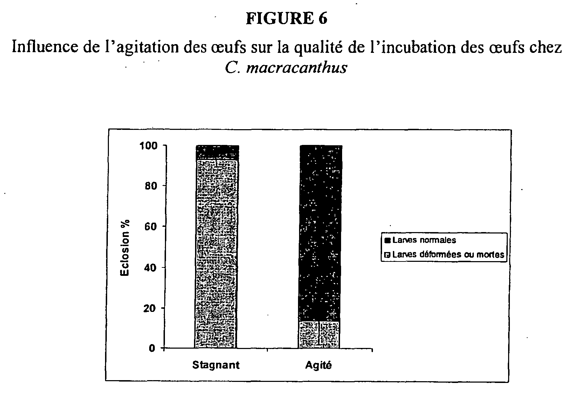

Les résultats sont donnés dans la figure 6.

L'incubation des oeufs en eau stagnante aboutit à un développement embryonnaire anormal, mis en évidence par une absence quasi-totale de mouvement des embryons en fin de développement, puis par la mortalité quasi-totale (plus de 90%) des larves à l'éclosion (Figure 6).

En revanche, incubés dans les mêmes conditions sur un plateau oscillant les oeufs se développent et éclosent normalement avec obtention de plus de 85% de larves normales i.e. actives et viables (Figure 6).

Lorsqu'ils sont placés dans l'eau après fécondation, les oeufs gonflent et développent un important espace périvitellin (le diamètre de l'oeuf augmente d'environ 300%), ce qui leur confère une flottabilité faiblement négative (un faible courant d'eau peut donc soulever les oeufs et les renvoyer en suspension dans la masse d'eau).

Des résultats similaires ont été obtenus avec un incubateur en forme d'entonnoir présentant une arrivée d'eau par le fond, permettant de maintenir les oeufs en mouvement dans un courant d'eau modéré (Figure 7). Dans ce type d'incubateur et dans des conditions de température adaptée, le pourcentage d'éclosion moyen obtenu atteint de 75,7 $\pm$ 6,2 % (n = 9 femelles), avec une proportion de larves déformées acceptable, en moyenne 17,0 $\pm$ 16,5 %.

*4.2.2. Influence de la température d'incubation*

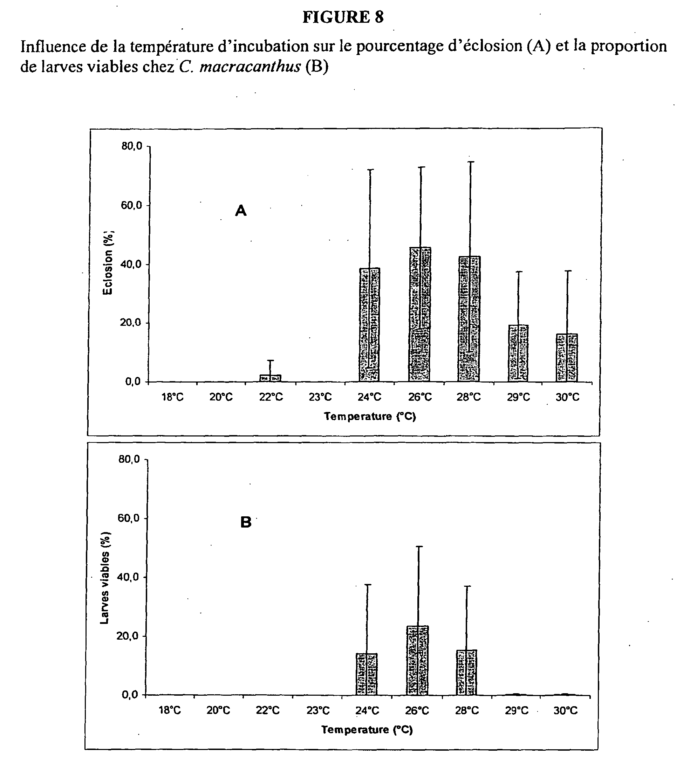

Les résultats sont donnés dans la figure 8.

Bien que *C. macracanthus* soit une espèce de poisson dont la distribution géographique se limite aux eaux douces tropicales des îles de Sumatra et de Bornéo en Indonésie, nos travaux démontrent une forte sensibilité des oeufs aux températures de l'eau de plus 28°C (auxquelles les oeufs de la plupart des poissons tropicaux se développent normalement), ainsi qu'aux températures inférieures à 24°C.

Les expériences réalisées de façon indépendante sur les oeufs de 9 femelles différentes issues des populations de Sumatra ou de Kalimantan (figure 7) montrent que :

- aucune éclosion n'est obtenue à 18°C comme à 20°C, le développement embryonnaire ne dépassant pas le stade Morula ;
- à 22-23°C, seules quelques rares éclosions de larves déformées (non viables) peuvent être obtenues ;
- à 29°C et 30°C, les pourcentages d'éclosion moyens obtenus n'excèdent pas 20%. De plus, les larves obtenues montrent des déformations notables et aucune d'entre elles n'est viable ;
- seule la plage de température allant de 24°C à 28°C a permis l'obtention de pourcentages d'éclosion moyens supérieur à 40% avec une forte proportion des larves normales et viables. Bien que les pourcentages d'éclosion et de larves normales obtenus à 24°C, 26°C et 28°C ne montrent pas de différences significatives au plan statistique, les valeurs les plus élevées sont obtenues à 26°C, au centre de cette plage thermique.

Une température de 26°C est donc recommandée pour l'incubation des oeufs de C. *macracanthus,* originaires tant des populations de Sumatra que de Bornéo.

**Exemple 5 : Conditions d'élevage larvaire**

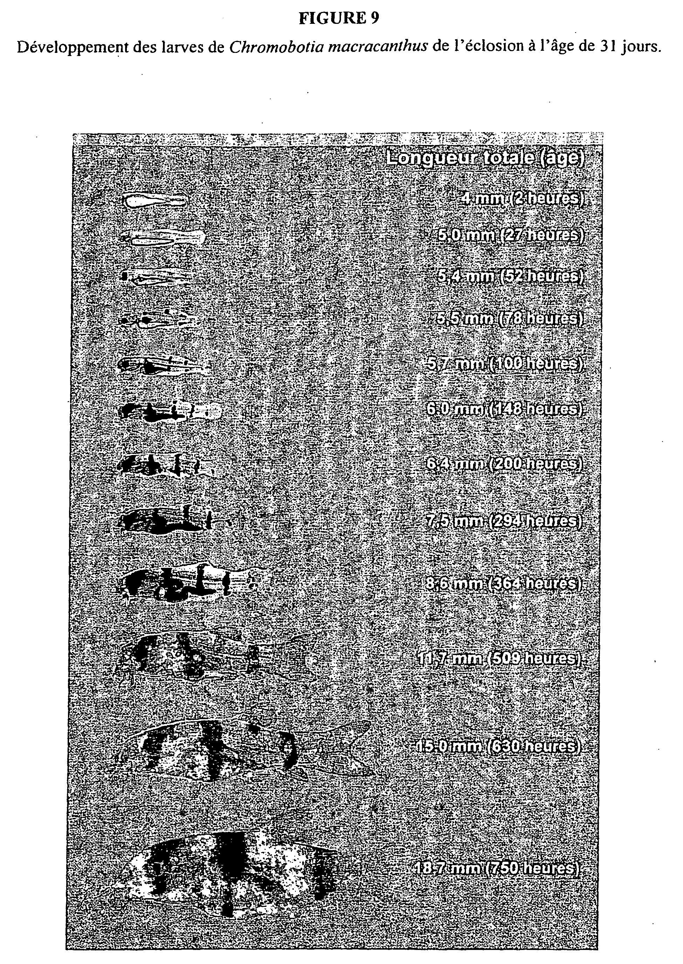

L'élevage larvaire n'est plus un problème, puisque les travaux des inventeurs ont montré que dans des conditions d'alimentation (nauplii d'*Artemia* en excès) et de qualité d'eau satisfaisante (concentration en oxygène dissous élevée, concentration en ammoniaque et nitrites faibles, pH neutre, température 28-30°C ....) des taux de survie compris entre 60 et 90% après un mois d'élevage Après deux mois d'élevage, les juvéniles peuvent être commercialisés (Figure 9).

**Revendications**

**1.** Procédé de production massive de poissons de l'ordre des Cypriniformes, notamment de la famille des Cobitidae en structure d'élevage en circuit fermé comprenant les étapes suivantes :

a) élevage dans des conditions dans lesquelles la luminosité est comprise entre 0 et 20 lux, des géniteurs mâles et femelles à une densité comprise entre 5 et 15 poissons par m$^3$, avantageusement entre 6 à 7 poissons par m$^3$, dans un bac d'élevage alimenté en eau dont la température est maintenue entre 30 et 32 °C pendant une période comprise entre 6 et 15 mois, avantageusement pendant 14 mois puis dont la température est diminuée à 25-29 °C, avantageusement 25-26 °C et maintenue à cette valeur pendant une période comprise entre 1 et 6 mois, avantageusement 3 mois, les géniteurs étant alimentés pendant toute la durée d'élevage le matin avec des aliments composés distribués à raison de 0,5 à 1 % de la biomasse des poissons et le soir avec des vers de terre représentant au moins 10 % de la biomasse des poissons, avantageusement entre 5 et 15 % de ladite biomasse,
b) sélection des génitrices femelles présentant un diamètre ovocytaire compris entre 1,00 et 1,32 mm,
c) sélection des géniteurs mâles chez lesquels le sperme s'écoule bien avec un massage abdominal,
d) traitement hormonal des génitrices femelles,

. soit par deux injections d'une combinaison de gonadolibérine purifiée ou sous forme d'analogues synthétiques et d'un composé anti-dopaminergique, lesdites injections étant séparées par un délai de 5 à 8 heures, avantageusement égal à 6 heures, les doses pour la première injection étant comprises entre 2 et 10 μg/kg, avantageusement égales à 8 μg/kg pour la gonadolibérine et ses analogues synthétiques et comprises entre 1 et 5 mg, avantageusement égales à 4 mg pour le composé antidopaminergique et les doses pour la deuxième injection étant comprises entre 8 et 12 μg/kg, avantageusement égales à 12 μg/kg pour la gonadolibérine purifiée ou sous forme d'analogues synthétiques et comprises entre 4 et 6 mg, avantageusement égales à 6 mg pour le composé antidopaminergique,
. soit par une première injection d'hormone gonadotropine chorionique humaine (hCG) à raison de 250 à 500 UI/kg, avantageusement à raison de 500 UI/kg suivie, 18 heures à 26 heures après, d'une seconde injection d'une combinaison de gonadolibérine purifiée ou sous forme d'analogue synthétique et d'un composé anti-dopaminergique à une dose comprise entre 4 et 12 μg/kg, avantageusement égale à 12 μg/kg pour la gonadotropine purifiée ou sous forme d'analogues synthétiques et à une dose comprise entre 2 et 6 mg/kg, avantageusement égale à 6 mg/kg pour le composés antidopaminergique,

e) traitement hormonal des géniteurs mâles par une injection d'une combinaison de gonadolibérine purifiée ou sous forme d'analogue synthétique et d'un composé anti-dopaminergique à une dose comprise entre 2 et 10 μg/kg, avantageusement égale à 8 μg/kg pour la gonadotropine purifiée ou sous forme d'analogues synthétiques et à une dose comprise entre 1 et 5 mg/kg, avantageusement égale à 4 mg/kg pour le composés antidopaminergique,
f) collecte des ovules par massage abdominal chez les génitrices femelles qui s'effectue avec un délai de latence moyen variable en fonction de la température de l'eau de maintenance des géniteurs et du traitement hormonal utilisé, selon les relations suivantes :

- pour le traitement avec deux injections successives de gonadolibérine associée à un antidopaminergique :

$$Y \text{ (Temps de latence en heure)} = 413,97 - 29,47\,X + 0,54\,X^2 \quad (R^2 = 0,670),$$

avec X= température en degrés Celsius
- pour le traitement avec une injection de hCG suivie d'une injection de gonadolibérine associée à un antidopaminergique :

$$Y \text{ (Temps de latence en heure)} = 771,51 - 54,08\,X + 0,96\,X^2 ; \quad (R^2 = 0,652),$$

avec X = température en degrés Celsius

g) collecte du sperme des géniteurs mâles 10 à 48 h après traitement hormonal, avantageusement 12 à 24 h, directement dans une solution d'immobilisation des spermatozoïdes, avantageusement une solution saline (NaCl 0,9%), et conservation au frais, avantageusement à 4 °C,
h) fécondation *in vitro* des oeufs,
i) incubation des oeufs sous légère agitation à une température comprise entre 24 et 28 °C, avantageusement à 26 °C jusqu'à éclosion,
j) recueil des larves,
k) élevage des larves jusqu'au stade juvénile.

2. Procédé selon la revendication 1 **caractérisé en ce que** les poissons sont des *Chromobotia macracanthus,* issus notamment des populations de Sumatra et de Bornéo (Kalimantan).

3. Procédé selon l'une quelconque des revendications 1 ou 2, **caractérisé en ce que** les analogues synthétiques de la gonadolibérine sont choisis dans le groupe comprenant GnRHa (avantageusement D-Arg6, Trp7, Leu8, Pro9, Net) ou LHRH-A et le composé antidopaminergique dans le groupe comprenant le dompéridone et le pimozide.

4. Procédé selon l'une quelconque des revendications 1 à 3, **caractérisé en ce que** l'eau utilisée est de l'eau recyclée circulant à un débit compris entre 80% et 100% de renouvellement en eau par heure du volume d'élevage.

5. Procédé selon l'une quelconque des revendications 1 à 4 **caractérisé en ce que** l'eau utilisée présente un pH compris entre 7,0 et 8,0, une conductivité comprise entre 100 et 300 μS/cm et **en ce que** la concentration en oxygène est comprise entre 6,0 et 8,0 mg/L.

6. Procédé selon l'une quelconque des revendications 1 à 5 **caractérisé en ce que** l'incubation des oeufs de l'étape i) est réalisée dans des conditions d'agitation modérée obtenues soit par agitation des oeufs dans un courant d'eau, avantageusement dans un incubateur en forme d'entonnoir comprenant une arrivée d'eau par le fond dans la partie étroite de l'entonnoir, ladite arrivée d'eau présentant un diamètre compris entre 20 et 30 mm pour permettre un débit d'entrée d'eau compris entre 0,3 L.mn$^{-1}$ et 2,3 L.mn$^{-1}$, soit dans des récipients en eau non renouvelée placés sur un plateau oscillant avec 56 à 88 oscillations par minute autour d'un angle d'une amplitude totale de 10 degrés (+5, -5).

**Patentansprüche**

1. Massenproduktionsverfahren für Fische der Ordnung der Karpfenartigen (Cypriniformes), insbesondere der Familie der Cobitidae, in einer Zuchtstruktur im geschlossenen Kreislauf, welches die folgenden Schritte umfasst:

a) Zucht, unter Bedingungen, unter denen die Beleuchtungsstärke zwischen 0 und 20 Lux liegt, von männlichen und weiblichen Elterntieren mit einer Dichte zwischen 5 und 15 Fischen pro m$^3$, vorteilhafterweise zwischen 6 und 7 Fischen pro m$^3$, in einem Zuchtbehälter, der mit Wasser gespeist wird, dessen Temperatur während eines Zeitraums, der zwischen 6 und 15 Monaten liegt und vorteilhafterweise 14 Monate beträgt, zwischen 30 und 32 °C gehalten wird, und dessen Temperatur danach auf 25-29 °C und vorteilhafterweise auf 25-26 °C verringert wird und während eines Zeitraums, der zwischen 1 und 6 Monaten liegt und vorteilhafterweise 3 Monate beträgt, bei diesem Wert gehalten wird, wobei die Elterntiere während der gesamten Zuchtdauer morgens mit Mischfuttermitteln gefüttert werden, die in einer Menge von 0,5 bis 1% der Biomasse der Fische verfüttert werden, und abends mit Regenwürmern, die wenigstens 10 % der Biomasse der Fische und vorteilhafterweise zwischen 5 und 15 % dieser Biomasse entsprechen,

b) Selektion der weiblichen Elterntiere, die einen Oocytendurchmesser zwischen 1,00 und 1,32 mm aufweisen,

c) Selektion der männlichen Elterntiere, bei denen das Sperma bei einer Abdominalmassage gut fließt,

d) hormonelle Behandlung der weiblichen Elterntiere,

- entweder durch zwei Injektionen einer Kombination von gereinigtem oder in Form von synthetischen Analoga vorliegendem Gonadoliberin und einer dopaminantagonistischen Verbindung, wobei die Injektionen durch einen Zeitraum von 5 bis 8 Stunden, vorteilhafterweise von 6 Stunden, getrennt sind, wobei die Dosen für die erste Injektion 2 bis 10 μg/kg, vorteilhafterweise 8 μg/kg, für das Gonadoliberin und dessen synthetische Analoga betragen und 1 bis 5 mg, vorteilhafterweise 4 mg, für die dopaminantagonistische Verbindung betragen und die Dosen für die zweite Injektion 8 bis 12 μg/kg, vorteilhafterweise 12 μg/kg, für das gereinigte oder in Form von synthetischen Analoga vorliegende Gonadoliberin betragen und 4 bis 6 mg, vorteilhafterweise 6 mg, für die dopaminantagonistische Verbindung betragen,

- oder durch eine erste Hormoninjektion von humanem Choriongonadotropin (hCG) in einer Menge von 250 bis 500 I.E./kg, vorteilhafterweise in einer Menge von 500 I.E./kg, 18 Stunden bis 26 Stunden später gefolgt von einer zweiten Injektion einer Kombination von gereinigtem oder in Form von synthetischen Analoga vorliegendem Gonadoliberin und einer dopaminantagonistischen Verbindung in einer Dosis von 4 bis 12 μg/kg, vorteilhafterweise von 12 μg/kg, für das gereinigte oder in Form von synthetischen Analoga vorliegende Gonadotropin und in einer Dosis von 2 bis 6 mg/kg, vorteilhafterweise von 6 mg/kg, für die dopaminantagonistische Verbindung,

e) hormonelle Behandlung der männlichen Elterntiere durch eine Injektion einer Kombination von gereinigtem oder in Form von synthetischen Analoga vorliegendem Gonadoliberin und einer dopaminantagonistischen Verbindung in einer Dosis von 2 bis 10 μg/kg, vorteilhafterweise von 8 μg/kg, für das gereinigte oder in Form von synthetischen Analoga vorliegende Gonadotropin und in einer Dosis von 1 bis 5 mg/kg, vorteilhafterweise von 4 mg/kg, für die dopaminantagonistische Verbindung,

f) Entnahme der Eizellen durch Abdominalmassage bei den weiblichen Elterntieren, welche mit einer mittleren Verzögerung durchgeführt wird, die in Abhängigkeit von der Temperatur des Wassers, in dem die Elterntiere gehalten werden, und von der angewendeten hormonellen Behandlung variabel ist, gemäß den folgenden Beziehungen:

- für die Behandlung mit zwei aufeinander folgenden Injektionen von Gonadoliberin in Verbindung mit einer dopaminantagonistischen Verbindung:

$$Y \text{ (Verzögerungszeit in Stunden)} = 413,97 - 29,47 \; X + 0,54 \; X^2; \; (R^2 = 0,670), \; mit \; X = Temperatur \; in \; Grad \; Celsius,$$

- für die Behandlung mit einer Injektion von hCG, gefolgt von einer Injektion von Gonadoliberin in Verbindung mit einer dopaminantagonistischen Verbindung:

$$Y \text{ (Verzögerungszeit in Stunden)} = 771,51 - 54,08 \; X + 0,96 \; X^2; \; (R^2 = 0,652), \; mit \; X = Temperatur \; in \; Grad \; Celsius,$$

g) Entnahme des Spermas der männlichen Elterntiere 10 bis 48 Stunden, vorteilhafterweise 12 bis 24 Stunden, nach der hormonellen Behandlung direkt in einer Immobilisierungslösung für die Spermien, vorteilhafterweise in einer Kochsalzlösung (NaCl 0,9 %), und Frischhalten, vorteilhafterweise bei 4 °C,

h) In-Vitro-Befruchtung der Eier,

i) Inkubation der Eier unter leichter Bewegung bei einer Temperatur von 24 bis 28 °C, vorteilhafterweise von 26 °C, bis zum Schlupf;

j) Einsammeln der Larven,

k) Aufzucht der Larven bis zum Jungfischstadium.

**2.** Verfahren nach Anspruch 1, **dadurch gekennzeichnet, dass** die Fische *Chromobotia macracanthus* sind, die insbesondere aus Populationen von Sumatra und Borneo (Kalimantan) stammen.

**3.** Verfahren nach einem der Ansprüche 1 oder 2, **dadurch gekennzeichnet, dass** die synthetischen Analoga von Gonadoliberin aus der Gruppe ausgewählt sind, die GnRHa (vorteilhafterweise D-Arg6, Trp7, Leu8, Pro9, Net) oder LHRH-A umfasst, und die dopaminantagonistische Verbindung aus der Gruppe, die Domperidon und Pimozid umfasst.

**4.** Verfahren nach einem der Ansprüche 1 bis 3, **dadurch gekennzeichnet, dass** das verwendete Wasser wiederaufbereitetes Wasser ist, das mit einer Durchflussmenge zirkuliert, die einer Erneuerung von 80 % bis 100 % des Wassers des Aufzuchtvolumens pro Stunde entspricht.

**5.** Verfahren nach einem der Ansprüche 1 bis 4, **dadurch gekennzeichnet, dass** das verwendete Wasser einen pH-Wert von 7,0 bis 8,0 und eine Leitfähigkeit von 100 bis 300 µS/cm aufweist, und dadurch, dass die Sauerstoffkonzentration 6,0 bis 8m0 m/l beträgt.

**6.** Verfahren nach einem der Ansprüche 1 bis 5, **dadurch gekennzeichnet, dass** die Inkubation der Eier in Schritt i) unter Bedingungen einer mäßigen Bewegung durchgeführt wird, die entweder durch Bewegung der Eier in einer Wasserströmung bewirkt wird, vorteilhafterweise in einem trichterförmigen Inkubator, der einen Wassereinlass über den Boden im engen Teil des Trichters umfasst, wobei dieser Wassereinlass einen Durchmesser zwischen 20 und 30 mm aufweist, um eine Wasserzuflussmenge von 0,3 l/min bis 2,3 l/min zu ermöglichen, oder in Behältern mit nicht erneuertem Wasser, die auf einer Platte angeordnet sind, welche mit 56 bis 88 Schwingungen pro Minute um einen Winkel mit einer Gesamtamplitude von 10 Grad (+5, -5) schwingt.

**Claims**

**1.** A method for the mass production of fish belonging to the order of Cypriniforms, notably of the Cobitidae family, in a closed circuit husbandry structure, including the following steps :

a) Raising in conditions wherein luminosity is between 0 - 20 Lux, of male and female spawners at a density between 5 - 15 fish per cubic meter, advantageously between 6 - 7 fish per cubic meter, in a husbandry pool fed with water with a temperature kept between 30 - 32 °C during a period between 6 - 15 months, advantageously during 14 months, whose temperature is then lowered to 25 - 29 °C, advantageously 25 - 26 °C and kept at this level during a period between 1 - 6 months, advantageously 3 months, the spawners being fed during the whole raising period, in the morning with mixed feed, distributed at a rate of 0.5 - 1 % of the fish biomass, and in the evening with earthworms representing at least 10 % of the fish biomass, advantageously between 5 - 15 % of said biomass,
b) selection of female spawners having an oocyte diameter between 1.00 and 1.32 mm,
c) selection of male spawners with whom the sperm flows easily by abdominal massage,
d) hormone treatment of female spawners,

• either with two injections of a combination of gonadoliberine, whether purified or in the form of synthetic analogues, and of an anti-dopaminergic compound, said injections being made at 5 - 8 hour intervals, advantageously 6 hours, the doses for the first injection being between 2 - 10 µg/kg, advantageously 8 µg/kg for gonadoliberine and its synthetic analogues, and being between 1 - 5 mg, advantageously 4 mg for the antidopaminergic compound, and the doses for the second injection being between 8 - 12 µg/kg, advantageously 12 µg/kg for the gonadoliberine, whether purified or in the form of synthetic analogues, and between 4 - 6 mg, advantageously 6 mg, for the antidopaminergic compound,
• or with a first injection of human chorionic gonadotropin hormone (hCG) at a rate of 250 - 500 IU/kg, advantageously of 500 IU/kg, this being followed, after 18 - 26 hours, by a second injection of a combination of gonadoliberine, whether purified or in the form of a synthetic analogue, and of an anti-dopaminergic compound, at a dose between 4 - 12 µg/kg, advantageously 12 µg/kg for gonadotropin, whether purified or in the form of synthetic analogues, and at a dose between 2 - 6 mg/kg, advantageously 6 mg/kg for the antidopaminergic compound,

e) hormone treatment of male spawners by injection of a combination of gonadoliberine, whether purified or in the form of a synthetic analogue, and of an anti-dopaminergic compound, at a dose between 2 - 10 µg/kg,

advantageously 8 μg/kg for the gonadotropin, whether purified or in the form of synthetic analogues, and at a dose between 1 - 5 mg/kg, advantageously 4 mg/kg, for the antidopaminergic compound,

f) collection of ova through abdominal massage of female spawners, which is carried out with an average lag time which may vary with the spawner maintenance water temperature and with the hormone treatment which is used, according to the following relationships :

- for the treatment with two successive injections of gonadoliberine as associated with an antidopaminergic agent:

$$Y \ (Lag \ time \ in \ hours) = 413.97 - 29.47 \ X + 0.54 \ X^2 \ (R^2 = 0.670),$$

with X = temperature in degrees Celsius

- for the treatment with a hCG injection followed by an injection of gonadoliberine as associated with an antidopaminergic agent:

$$Y \ (Lag \ time \ in \ hours) = 771.51 - 54.08 \ X + 0.96 \ X^2 \ ; \ (R^2 = 0.652),$$

with X = temperature in degrees Celsius

g) collection of sperm from male spawners 10 - 48 h after hormone treatment, advantageously 12 - 24 h, directly into a spermatozoid immobilization solution, advantageously a saline solution (NaCl 0.9%), and keeping at a cold temperature, advantageously at 4 °C,

h) *in vitro* fertilization of the eggs,

i) incubation of the eggs under slight agitation at a temperature between 24 - 28 °C, advantageously 26 °C until hatching,

j) collection of the larvae,

k) raising of larvae until juvenile stage.

2. A method according to claim 1, **characterized in that** the fish are *Chromobotia macracanthus,* notably from Sumatra and Borneo (Kalimantan) populations.

3. A method according to claim 1 or 2, **characterized in that** the synthetic analogues of gonadoliberine are chosen among the group comprising GnRHa (advantageously D-Arg6, Trp7, Leu8, Pro9, Net) or LHRH-A and the anti-dopaminergic compound among the group including domperidone and pimozide.

4. A method according to any claims 1 to 3, **characterized in that** the water which is used is recycled water circulating at a flow rate between 80% - 100% renewal of the water per hour of the raising volume.

5. A method according to any claims 1 to 4, **characterized in that** the water which is used has a pH between 7.0 and 8.0, a conductivity between 100 - 300 μS/cm, and **in that** the oxygen concentration is between 6.0 - 8.0 mg/L.

6. A method according to any claims 1 to 5, **characterized in that** the incubation of the eggs from Step i) is carried out under moderate agitation conditions, which are obtained either by agitating the eggs in a flow of water, advantageously in a funnel-shaped incubator including a bottom water inlet in the narrow part of the funnel, said water inlet having a diameter between 20 - 3 0 mm to allow a water inlet flow rate between 0.3 L.mn$^{-1}$ - 2.3 L.mn$^{-1}$, or in non renewed water containers which are placed onto an oscillating tray oscillating at 56 - 88 oscillations per minute around an angle having a total amplitude of 10 degrees (+5, -5).

EP 2 262 361 B1

## FIGURE 1

Evolution du pourcentage de femelles en cours de vitellogénèse (stade 3) et en fin de vitellogénèse (stade 4) de *C. macracanthus* provenant de Sumatra (**A**) et de Kalimantan (**B**) maintenues en captivité en circuit fermé

16

## FIGURE 2

Evolution du pourcentage de mâles en cours de maturation sexuelle (stade 1) et de males (stade 2 et 3) de *C. macracanthus* provenant de Sumatra (**A**) et de Kalimantan (**B**) maintenus en captivité en circuit fermé

**FIGURE 3**

Evolution moyenne du diamètre modal ovocytaire de 2 femelles de *C. macracanthus* isolées à 25-26°C et de 2 autres à 30-31°C.

FIGURE 4

Pourcentage d'ovulation obtenu après traitement hormonal en fonction du diamètre modal initial des ovocytes.

## FIGURE 5

Relation entre le temps de latence après traitement hormonal et la température de l'eau de stabulation des géniteurs de *Chromobotia macracanthus*

A : femelles traitées avec deux injections successives de Ovaprim

B : femelles traitées avec une injection de hCG suivie d'une injection de Ovaprim.

**FIGURE 6**

Influence de l'agitation des œufs sur la qualité de l'incubation des œufs chez
*C. macracanthus*

**FIGURE 7**

Modèle d'incubateur en forme d'entonnoir et en fibre de verre

*diametre* 35 cm

10 cm
3 cm

30 cm

30°

Réduction P.V.C
1 pouce*→ 0,5 pouce
*1 pouce = 2,54 cm

## FIGURE 8

Influence de la température d'incubation sur le pourcentage d'éclosion (A) et la proportion de larves viables chez *C. macracanthus* (B)

# FIGURE 9

Développement des larves de *Chromobotia macracanthus* de l'éclosion à l'âge de 31 jours.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SATYANI D.** *Journ. Penel. Perik. Indon.,* 2004, vol. 10 (5), 55-59
- **SATYANI D. ; H. MUNDRIYANTO ; T. KADARINI DAN KUSDIARTI.** Prosiding Seminar Nasional dan Konggres Biologi. *Fakultas Biologi UGM,* 2005, vol. XIII, 435-438
- **SUBANDIYAH S.** Prosiding Seminar Nasional dan Konggres Biologi. *Fakultas Biologi UGM,* 2005, vol. XIII, 290-293
- **SUBANDIYAH S ; DAN D. SATYANI.** Prosiding Seminar Biologi menuju Millenium. *Fakultas Biologi UGM,* 2004, vol. III, 127-136
- **SATYANI D. ; CHUMAIDI DAN KUSDIARTI.** *Pros. Sem. Nas.,* 2006
- **SUBANDIYAH S.** *Prosiding Seminar nasional Perikanan Indonesia,* 2005, 185-192
- **CHUMAIDI.** Prosiding Sem Nasional Tahunan Hasil Penel. *Perik. dan Kelautan. UGM,* 2005, 249-254
- **EFFENDI I. ; T. PRASETYA ; A.O. SUDRAJAT ; N. SUHENDA ; K. SUMAWIDJAJA.** *Jurnal Akuakultur Indonesia,* 2003, vol. 2 (2), 51-54
- **SATYANI D. ; T. KADARINI ; DAN O. KOMARUDIN.** *Joum. Penel. Perik. Indon.,* 1999, vol. II (4), 37-42
- **SUBAGJA J. ; O. KOMARUDIN ; DAN J. EFFENDI.** *Jurn. Penel. Perik. Indon.,* 1997, vol. III (2), 10-17